(19) Europäisches Patentamt
European Patent Office
Office européen des brevets

(11) **EP 4 108 307 A2**

(12) **EUROPEAN PATENT APPLICATION**

(43) Date of publication:
**28.12.2022 Bulletin 2022/52**

(21) Application number: **22180694.6**

(22) Date of filing: **23.06.2022**

(51) International Patent Classification (IPC):
**B01D 21/00** (2006.01)     **B01D 21/24** (2006.01)
**B01D 21/30** (2006.01)     **C12M 1/26** (2006.01)
**C12M 1/02** (2006.01)     **C12M 1/00** (2006.01)
**C12M 1/34** (2006.01)

(52) Cooperative Patent Classification (CPC):
**B01D 21/00; B01D 21/0087; B01D 21/24;
B01D 21/30; C12M 27/00; C12M 29/00;
C12M 33/00; C12M 33/22; C12M 41/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.06.2021   JP 2021104556**

(71) Applicant: **Yokogawa Electric Corporation
Musashino-shi, Tokyo 180-8750 (JP)**

(72) Inventors:
• **NAMATAME, Tetsushi
  Musashino-shi, Tokyo, 180-8750 (JP)**
• **HASHIMOTO, Himuro
  Musashino-shi, Tokyo, 180-8750 (JP)**

(74) Representative: **Hoffmann Eitle
Patent- und Rechtsanwälte PartmbB
Arabellastraße 30
81925 München (DE)**

(54) **DEVICE FOR LIQUID PROCESSING IN CELL CULTURE PROCESS**

(57)     The present disclosure relates to a device for liquid processing in the process of cell culture, comprises: a settling tank which is configured to store a cell culture fluid from a bioreactor; a communicating tube that allows the bioreactor to communicate with the settling tank; a first excretory tube for discharging a cell concentrate, which is connected to the settling tank; a second excretory tube for discharging a supernatant, which is connected to the settling tank; a pump; and a control unit. In such device, after the cell culture fluid is separated into the supernatant and the cell concentrate by the gravitational settling in the settling tank, the control unit is configured to control the pump to (a) return the supernatant to the bioreactor through the communicating tube and harvest the cell concentrate through the first excretory tube and/or (b) harvest the supernatant through the second excretory tube and return the cell concentrate to the bioreactor through the communicating tube.

Fig. 2

**EP 4 108 307 A2**

## Description

BACKGROUND

Technical Field

The present disclosure relates to a method for separation and/or concentration of cells in a process of cell culture and a device for use in liquid processing therefor. The present disclosure also relates to a method for producing a target product via cell culture, and a cell culture system.

Background Art

Production of bio-pharmaceuticals includes a step of culture for producing a target product via biological reactions and a step of purification by removing contaminants generated in the step of culture to increase a degree of purity of the target product. In a step of culture for representative bio-pharmaceuticals; i.e., antibody pharmaceuticals, animal cells, such as CHO cells, have often been used. However, animal cells are likely to be influenced by a culture environment, and the yield and the quality of a target product are adversely influenced when an adequate environment is not maintained. Examples of factors that deteriorate the culture environment include mechanical stress caused by stirring or gas aeration, depletion of nutrients or oxygen, and accumulation of waste products such as lactic acid or ammonia generated by cells. Accordingly, a method of production in which substances required by cells are supplemented during culture while fundamental environmental factors, such as the dissolved oxygen (DO) level, pH, temperature, and a stirring speed of a cell culture fluid, are regulated is employed. Examples of substances supplemented during culture include nutrients contained in a medium and substances that increase the cell growth speed or production speed referred to as potentiators.

Methods of culture in which substances are supplemented during culture include continuous culture, perfusion culture, and fed-batch culture. A method of continuous culture is performed by removing a part of a cell culture fluid containing cells during culture and introducing a fresh cell culture fluid in an amount equivalent to the amount of the removed solution.

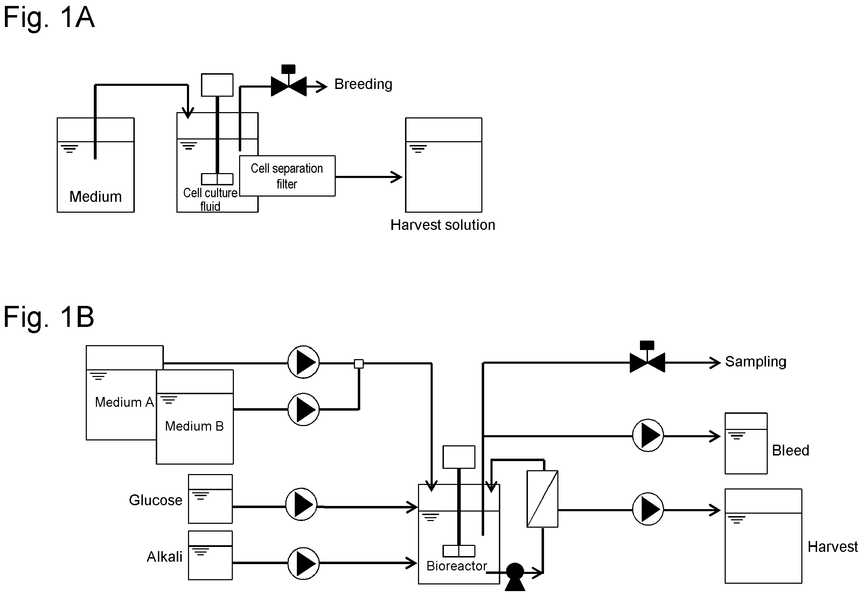

A method of perfusion culture is performed by selectively removing a part of a liquid component remaining after cell separation during culture and introducing a fresh cell culture fluid in an amount equivalent to the amount of the removed solution. In the case of continuous culture and perfusion culture, a constant culture environment can easily be maintained, and stable production can be performed. In recent years, accordingly, such methods are often applied to research and development or production. In production of antibody pharmaceuticals, in particular, the cell density can easily be maintained at a high level in perfusion culture as shown in Fig. 1A and 1B, and a production speed per time can be maintained at a high level. Thus, many studies thereon have been reported (e.g., JP 2019-213497 A, WO 2018/159847 A, and WO 2015/012174 A).

When perfusion culture is initiated, a medium and cells are introduced into a stirred-tank bioreactor (initial cell density: approximately $2 \times 10^5$ to $1 \times 10^6$ cells/ml), and cell growth proceeds when temperature, pH, and DO in the bioreactor are adequately maintained. When cell growth proceeds to a certain extent (2 or 3 days after the initiation of culture), media are continuously supplied to the bioreactor, and a harvest solution (a cell-separated solution) in an amount equivalent to the amount supplied is harvested. Since the amount supplied is equivalent to the amount harvested, the volume of the culture solution can be maintained at a constant level in the bioreactor. Cells remain in a bioreactor by the action of a hollow fiber filter of a cell separation apparatus, which is referred to as tangential flow filtration (TFF) or alternating tangential flow (ATF) filtration. Thus, cell growth continues in the bioreactor after the initiation of perfusion. When the number of cells is increased to a given density (approximately $1 \times 10^7$ to $1 \times 10^8$ cells/ml) or more, regulation of DO or adjustment of nutrients as described above becomes difficult, which lead to the deteriorated culture environment. In general, accordingly, a means for discharging excess cells to the outside of the bioreactor (i.e., bleeding) is employed to adjust the cell density within an adequate range by removing the cell culture fluid when the level of cell growth reaches the target level. In general, a perfusion rate (a proportion of the amount of the medium introduced relative to the volume of the cell culture fluid per day) is approximately 1 to 3 VVD, the amount of the bleed liquid discharged is approximately 0.2 to 0.5 VVD, and operation is continued for approximately 4 to 12 weeks.

SUMMARY OF THE INVENTION

A process of cell culture, such as perfusion culture, had problems as described below. When performing bleeding of discharging cells as described above, specifically, a discharged bleed liquid contains a target product, such as an antibody. When bleeding is performed many times, accordingly, the efficiency for harvesting a product is decreased. While it is possible to remove cells from the discharged bleed liquid and harvest a target product therefrom, disadvantageously, a procedure or a constitution of an apparatus becomes complicated. It is accordingly necessary to minimize the amount of the bleed liquid to be discharged, and cell growth may be terminated while maintaining the cell production speed after the target cell density is achieved. At present, however, there is no technique to overcome such problems, and, as a result of bleeding, a cell culture fluid in an amount of 20% to 30% relative to the entire cell culture fluid is discharged from the culture. In case of cells that grow rapidly, it is necessary to subject a cell culture fluid in an amount of approximately 60% relative to the entire cell culture fluid to bleeding in order to regulate the cell density.

Also, the culture environment in the bioreactor may be deteriorated when debris derived from dead cells or aggregated cells are increased. That is, cell death occurs at a constant rate even if an adequate environment is maintained in the bioreactor. Since it is impossible to selectively separate and discharge dead cells, the number of dead cells increases with the elapse of time. While the speed or extent of dead cell degradation varies depending on the aspect of death, such as necrosis or apoptosis, cells are degraded into debris with the elapse of time. While cell-separation filters with a pore size of approximately 0.2 $\mu$m have often been employed, particles larger than such pore size are not discharged as a harvest solution, and debris are thus increased in the bioreactor. While a certain amount of debris is discharged upon bleeding, many debris remain in the bioreactor. As the culture proceeds, accordingly, dead-cell-derived debris increase, and the culture environment is deteriorated. In addition, cell aggregation or cell enlargement incurs similar problems, but there is no technique to separate and discharge such abnormal cells. With the elapse of time, accordingly, the environment in the bioreactor is deteriorated gradually. In case of cells that grow particularly rapidly, it is difficult to stably perform culture over a long period exceeding 1 month.

As a result of concentrated studies, the present inventors have found that cells could be separated and/or concentrated in a simple and efficient manner by providing a settling tank in a tube through which a cell culture fluid is removed from the bioreactor as bleeding in a process of cell culture, separating the cell concentrate from the supernatant by the gravitational settling in the settling tank, harvesting either one of the cell concentrate or the supernatant, and returning the other to the bioreactor. It was found that the amount of a bleed-processed liquid could be reduced or debris could be selectively discharged by separation and/or concentration of cells as described above. It was also found that such processing and means could be applied to separation and/or concentration of cells in the process of cell culture.

Specifically, the present disclosure provides [1] to [15] described below.

[1] A device for use in liquid processing in a process of cell culture, comprising:

a settling tank which is configured to store a cell culture fluid from a bioreactor;
a communicating tube that allows the bioreactor to communicate with the settling tank;
a first excretory tube for discharging a cell concentrate, which is connected to the settling tank;
a pump; and
a control unit,
wherein, after the cell culture fluid is separated into a supernatant and the cell concentrate by the gravitational settling in the settling tank, the control unit is configured to control the pump to return the supernatant to the bioreactor through the communicating tube and to harvest the cell concentrate through the first excretory tube.

[2] The device for use according to [1], which further comprises a second excretory tube for discharging the supernatant, which is connected to the settling tank,
wherein the control unit is configured to further control the pump to harvest the supernatant through the second excretory tube and to return the cell concentrate to the bioreactor through the communicating tube.

[3] A device for use in liquid processing in a process of cell culture, comprising:

a settling tank which is configured to store a cell culture fluid from a bioreactor;
a communicating tube that allows the bioreactor to communicate with the settling tank;
a second excretory tube for discharging a supernatant, which is connected to the settling tank;
a pump; and
a control unit,
wherein, after the cell culture fluid is separated into the supernatant and a cell concentrate by the gravitational settling in the settling tank, the control unit is configured to control the pump to harvest the supernatant through the second excretory tube and to return the cell concentrate to the bioreactor through the communicating tube.

[4] The device for use according to [3], which further comprises a first excretory tube for discharging the cell concentrate, which is connected to the settling tank,
wherein the control unit is configured to further control the pump to return the supernatant to the bioreactor through the communicating tube and to harvest the cell concentrate through the first excretory tube.

[5] The device for use according to [2] or [4], which further comprises a flow path switching valve that connects the first excretory tube to the second excretory tube,

wherein the control unit is configured to control switching of the flow path switching valve, forward operation and backward operation of the pump, and migration of the supernatant and the cell concentrate.

[6] The device for use according to any of [1] to [5],

wherein the communicating tube is connected to the upper part of the settling tank,
the first excretory tube is connected to the lower part or the bottom of the settling tank, and/or
the second excretory tube is connected to a site that is in the upper part of the settling tank and is not in contact with the communicating tube.

[7] The device for use according to any of [1] to [6], wherein the settling tank is provided in the bioreactor.

[7-1] The device for use according to any of [1] to [7], wherein the settling tank comprises an inclined plate.

[8] The device for use according to any of [1] to [7], wherein the settling tank comprises a temperature regulation mechanism.

[8-1] The device for use according to any of [1] to [7], wherein the settling tank comprises a means for oxygen incorporation.

[8-2] The device for use according to any of [1] to [7], which further comprises a third excretory tube and a check valve in the lower part or at the bottom of the settling tank.

[8-3] The device for use according to any of [1] to [7], wherein the communicating tube comprises a check valve.

[9] A cell culture system, comprising:

a bioreactor is configured to accommodate a cell culture fluid containing cells;
a stirring device; and
the device according to any of [1] to [8].

[9-1] The system according to [9], which further comprises a tank which is configured to store a target product harvested from the cell culture fluid.

[10] A method for separating and/or concentrating cells in a process of cell culture, comprising:

a step of transferring a cell culture fluid from a bioreactor to a settling tank;
a step of separating the cell culture fluid into a supernatant and a cell concentrate by the gravitational settling in the settling tank; and
a step of returning the supernatant to the bioreactor and harvesting the cell concentrate.

[11] The method according to [10], wherein, on the basis of the duration of a sequence, and the amount of the cell culture fluid transferred per unit time or the number of cells harvested per unit time, at least one factor is calculated, which is selected from the group consisting of the latency of the gravitational settling, the time for transfer of the cell culture fluid, the time for transfer of the supernatant and/or the cell concentrate, the amount of the cell concentrate harvested, and the amount of the supernatant returned.

[12] The method according to [10] or [11], wherein, when returning the supernatant to the bioreactor, the speed for returning the supernatant is controlled so as to suppress the cells in the cell concentrate from floating.

[13] A method for separating and/or concentrating cells in a process of cell culture, comprising:

a step of transferring a cell culture fluid from a bioreactor to a settling tank;
a step of separating the cell culture fluid into a supernatant and a cell concentrate by the gravitational settling in the settling tank; and
a step of harvesting the supernatant and returning the cell concentrate to the bioreactor.

[14] The method according to [13], wherein, on the basis of the duration of a sequence, and the amount of the cell culture fluid transferred per unit time or the number of cells harvested per unit time, at least one factor is calculated, which is selected from the group consisting of the latency of the gravitational settling, the time for transfer of the cell culture fluid, the time for transfer of the supernatant and/or the cell concentrate, the amount of the supernatant harvested, and the amount of the cell concentrate returned.

[15] A method for producing a target product via cell culture, comprising:

a step of culturing cells that produce a target product in a bioreactor;
a step of implementing the method according to any of [10] to [14]; and
a step of harvesting the target product from the cell culture fluid in the bioreactor.

[15-1] The method according to [15], wherein the cells are mammalian animal cells.

[15-2] The method according to [15], wherein the target product comprises a target protein.

According to the present disclosure, excess cells generated in a process of cell culture can be separated and/or concentrated in a simple and efficient manner, the amount of normal cells wasted can be reduced, and unnecessary substances such as debris can be removed in an efficient manner. Thus, the present disclosure is useful in the field of, for example, cell culture and production of bio-pharmaceuticals via cell culture.

Brief Description of the Drawings

Figs. 1A and 1B show schematic diagrams of common perfusion culture (Fig. 1A) and a constitutional example of a cell culture system for common perfusion culture (Fig. 1B).

Fig. 2 schematically shows an embodiment of a device for liquid processing.

Fig. 3 schematically shows an example of a device for liquid processing provided in a bioreactor.

Figs. 4A and 4B show a flow example of a concentration sequence of the method of cell separation and/or concentration (Fig. 4A) and a flow example of a clarification sequence (Fig. 4B).

Figs. 5A and 5B show a flow example of a concentration sequence and a clarification sequence ST1 (Fig. 5A) and a schematic diagram of an operation example of a device for liquid processing (Fig. 5B).

Figs. 6A and 6B show a flow example of a concentration sequence and a clarification sequence ST2 (Fig. 6A) and a schematic diagram of an operation example of a device for liquid processing (Fig. 6B).

Fig. 7 shows a chart demonstrating the correlation between the latency and the number of cells settled when a cell culture fluid is separated by the gravitational settling in a settling tank.

Figs. 8A and 8B show a flow example of a concentration sequence ST3 (Fig. 8A) and a schematic diagram of an operation example of a device for liquid processing (Fig. 8B).

Figs. 9A and 9B show a flow example of a concentration sequence ST4 (Fig. 9A) and a schematic diagram of an operation example of a device for liquid processing (Fig. 9B).

Figs. 10A and 10B show a flow example of a clarification sequence ST3' (Fig. 10A) and a schematic diagram of an operation example of a device for liquid processing (Fig. 10B).

Figs. 11A and 11B show a flow example of a clarification sequence ST4' (Fig. 11A) and a schematic diagram of an operation example of a device for liquid processing (Fig. 11B).

Figs. 12A and 12B show modification examples of a device for liquid processing.

Figs. 13A to 13C show modification examples of a device for liquid processing.

Fig. 14 shows a chart demonstrating changes in cell distribution after cell settling in a clarified liquid layer in the settling tank.

DETAILED DESCRIPTION

The present disclosure relates to a method for separating and/or concentrating cells in a process of cell culture, which may be employed in bleeding, and a device for liquid processing used therefor. The term "bleeding (bleed processing)" used herein refers to a process of removing a cell culture fluid containing some of the cells increased in a bioreactor in a process of cell culture, and, in particular, in a process of perfusion culture, so as to discharge excess cells from the bioreactor. The cell culture fluid thus removed is referred to as a "bleed-processed liquid."

In the present disclosure, a settling tank may be provided in a tube through which a cell culture fluid is removed from a bioreactor (e.g., a tube through which a cell culture fluid is removed from a bioreactor as bleeding), a cell concentrate may be separated from a supernatant by the gravitational settling in the settling tank, either of the cell concentrate or supernatant is harvested, the other is returned to the bioreactor, and cells may thus be separated and/or concentrated. Thus, normal cells may be separated from abnormal cells (enlarged cells or aggregated cells) and debris, abnormal cells and debris may be harvested, and/or normal cells may selectively be returned to the bioreactor.

When discharging a cell concentrate (i.e., a concentration sequence), aggregated or enlarged cells can be selectively removed, and the amount of a cell culture fluid to be discharged can be reduced via concentration. When the present disclosure is applied to bleeding, for example, the amount of bleeding can be reduced via concentration of a bleed liquid. Specifically, the cell density in the cell culture fluid discharged by bleeding can be increased, and the amount of bleeding so as to maintain the cell density to a constant level can be suppressed. In such a case, aggregated or enlarged cells can be preferentially removed, and the effects of maintaining a good culture environment over a long period of time can also be attained.

When discharging a supernatant (i.e., a clarification sequence), debris can be selectively removed from a cell culture fluid. When the present disclosure is applied to bleeding, for example, debris can be selectively discharged via clarification of a bleed liquid, and a good culture environment can be maintained over a long period of time.

Hereafter, specific aspects and embodiments of the present disclosure are described in detail with reference to the drawings. It should be noted that the present disclosure is not limited to such specific aspects and embodiments and that a person skilled in the art would readily modify the constitution within the idea or spirit of the present disclosure.

(A device for liquid processing)

An aspect of the present disclosure provides a device for liquid processing in a process of cell culture, comprising:

a settling tank which is configured to store a cell culture fluid from a bioreactor;
a communicating tube that allows the bioreactor to communicate with the settling tank;
a first excretory tube for discharging a cell concentrate, which is connected to the settling tank;
a pump; and
a control unit.

In such device, after the cell culture fluid is separated into a supernatant and the cell concentrate by the gravitational settling in the settling tank, the control unit is configured to control the pump to (a) return the supernatant to the bioreactor through the communicating tube and harvest the cell concentrate through the first excretory tube. In an embodiment, a device for liquid processing may further comprise a second excretory tube for discharging a supernatant, which is connected to the settling tank, and the control unit is configured to further control the pump to (b) harvest the supernatant through the second excretory tube and return the cell concentrate to the bioreactor through the communicating tube.

Another aspect of the present disclosure provides a device for liquid processing in a process of cell culture, comprising:

a settling tank which is configured to store a cell culture fluid from a bioreactor;
a communicating tube that allows the bioreactor to communicate with the settling tank;
a second excretory tube for discharging a supernatant, which is connected to the settling tank;
a pump; and
a control unit.

In such device, after the cell culture fluid is separated into a supernatant and the cell concentrate by the gravitational settling in the settling tank, the control unit is configured to control the pump to (b) harvest the supernatant through the second excretory tube and return the cell concentrate to the bioreactor through the communicating tube. In an embodiment, a device for liquid processing may further comprise a first excretory tube for discharging a cell concentrate, which is connected to the settling tank, and the control unit is configured to further control the pump to (a) return the supernatant to the bioreactor through the communicating tube and harvest the cell concentrate through the first excretory tube.

A further aspect of the present disclosure provides a device for liquid processing in a process of cell culture, comprising:

a settling tank which is configured to store a cell culture fluid from a bioreactor;
a communicating tube that allows the bioreactor to communicate with the settling tank;
a first excretory tube for discharging a cell concentrate, which is connected to the settling tank;
a second excretory tube for discharging a supernatant, which is connected to the settling tank;
a pump; and
a control unit.

In such device, after the cell culture fluid is separated into the supernatant and the cell concentrate by the gravitational settling in the settling tank, the control unit is configured to control the pump to:

(a) return the supernatant to the bioreactor through the communicating tube and harvest the cell concentrate through the first excretory tube; and/or
(b) harvest the supernatant through the second excretory tube and return the cell concentrate to the bioreactor through the communicating tube.

In an embodiment, the control unit is configured to control the pump to (a) return the supernatant to the bioreactor through the communicating tube and harvest the cell concentrate through the first excretory tube, and (b) harvest the supernatant through the second excretory tube and return the cell concentrate to the bioreactor through the communicating tube.

The device for liquid processing according to the present disclosure can be used for processings, such as bleeding, cell separation, cell isolation, or cell harvesting, in a process of cell culture.

For example, a control unit may be configured to control forward operation and backward operation of a pump to control migration of the supernatant and the cell concentrate. A device for liquid processing comprises at least one pump and it may comprise a plurality of pumps. For example, a pump may control migration of supernatants and cell concentrates in all of the communicating tube, the first excretory tube, and/or the second excretory tube. Alternatively, a plurality of pumps may control migration of liquids in 1 or 2 of the communicating tube, the first excretory tube, and/or the second excretory tube. When a pump controls liquid migration in 2 or more tubes, the pump is configured to control liquid migration in such tubes with the use of flow path switching valves.

In an embodiment, accordingly, a device for liquid processing may further comprise a flow path switching valve. For example, a device for liquid processing may comprise a flow path switching valve that connects the first excretory tube to the second excretory tube. This can reduce the number of pumps to be provided, and a constitution of the device can be simplified. In the process of perfusion culture, it is necessary to simultaneously control a plurality of pumps that adjust the flow rates of a plurality of inflow/outflow lines for a cell culture fluid as shown in Fig. 1B. By reducing the number of pumps with the use of a flow path switching valve, accordingly, operation of the device and the system can be simplified. By the operation of the flow path switching valve, in addition, foams can be prevented from remaining in the settling tank. In the presence of a flow path switching valve, the control unit may be configured to control switching of the flow path switching valve, forward operation and backward operation of the pumps, and migration of the supernatant and the cell concentrate.

The settling tank may be formed to have a configuration, a size, and a material suitable for storing a cell culture fluid and separating a supernatant from a cell concentrate by the gravitational settling. The settling tank can be made to be a cylindrical or polygonal form or other, and the bottom may have a conical form, so that the cell concentrate may easily be settled and accumulated. A size (volume) of the settling tank can be adequately determined in accordance with, for example, a size of the bioreactor, the amount of the cell culture fluid to be stored (e.g., the amount of the bleed-processed liquid), the processing (e.g., bleeding) speed, or types, sizes, and growth rates of cells to be cultured. The settling tank can be made of any materials that are generally used for a cell culture vessel without particular limitation.

As a communicating tube, a first excretory tube, a second excretory tube, and a pump, relevant components that are generally used in cell culture can be used, and a person skilled in the art can adequately select such components. A control unit may be configured to control migration of at least one of a cell culture fluid, a supernatant, and a cell concentrate in a device for liquid processing. For example, the control unit may be configured to control inflow of an adequate amount of a cell culture fluid from a bioreactor to a settling tank, harvesting of a cell concentrate or return thereof to a bioreactor, and/or harvesting of a supernatant or return thereof to a bioreactor. Any control means that is known in the art can be used as the control unit. For example, a computer can be used.

In some embodiments, a communicating tube may be connected to the upper part of a settling tank. In some embodiments, a first excretory tube is connected to the lower part or the bottom of the settling tank. In some other embodiments, it may be connected to the bottom of the settling tank. In some embodiments, a second excretory tube may be connected to a site that is in the upper part of the settling tank and is not in contact with a communicating tube.

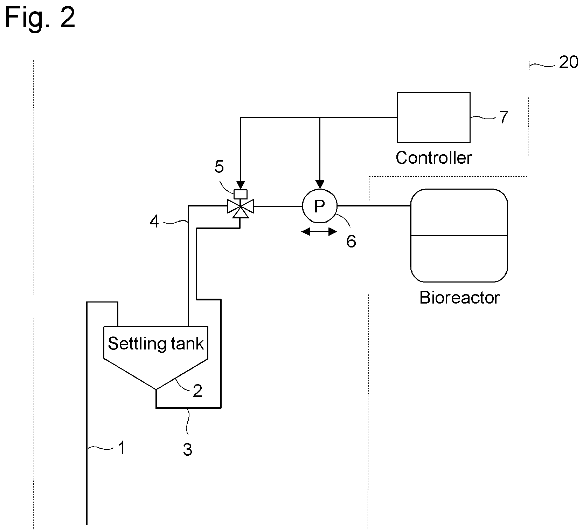

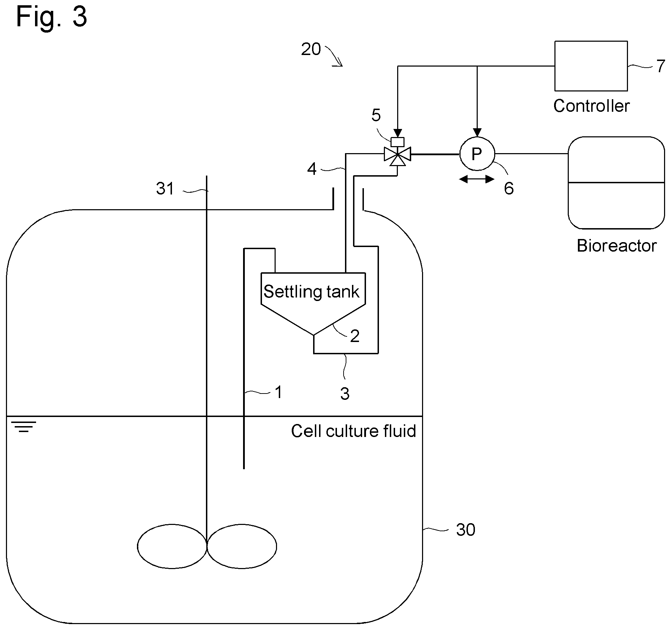

Hereafter, the constitution of the device for liquid processing according to the present disclosure is described with reference to specific embodiments. Fig. 2 schematically shows an embodiment of the device for liquid processing and Fig. 3 schematically shows an example of the device for liquid processing provided in a bioreactor.

The device 20 for liquid processing shown in Fig. 2 comprises the communicating tube 1 that allows a bioreactor to communicate with the settling tank 2, the settling tank 2, the excretory tube 3 for a concentrate, the excretory tube 4 for a supernatant, the flow path switching valve 5, the liquid transfer pump 6, and the controller 7. Fig. 3 shows an example of the device 20 provided in the bioreactor 30 comprising the stirring rod 31.

In this embodiment, the device 20 for liquid processing transfers a cell culture fluid from the bioreactor 30 to the settling tank 2 through the communicating tube 1, separates the cell culture fluid into a supernatant and a cell concentrate by the gravitational settling in the settling tank 2, (a) returns the supernatant to the bioreactor 30 through the communicating tube 1, and harvests the cell concentrate through the excretory tube 3. After the device 20 for liquid processing separates the cell culture fluid into a supernatant and a cell concentrate by the gravitational settling in the settling tank 2, alternatively, the device 20 (b) harvests the supernatant through the excretory tube 4 and returns the cell concentrate to the bioreactor 30 through the communicating tube 1. In this embodiment, the device is constituted to be capable of performing both (a) and (b) described above, but the device may be constituted to selectively perform either (a) or (b). The pump 6 is a liquid-transfer pump to allow liquid in a tube and a settling tank to migrate, and the controller 7 controls at least the pump 6. When a pump is connected to a plurality of tubes as shown in the figure, the controller also controls the flow path switching valve 5. In the presence of a plurality of pumps, in contrast, the controller 7 controls such pumps.

Each component of the device 20 for liquid processing may be designed to adjust the cell density in the bleed liquid by, for example, providing a settling tank in a bleed line (i.e., a line that performs bleeding, more specifically, a line that discharges cells).

The volume (size) of the settling tank 2 can be determined in view of, for example, the cell settling speed in the settling tank 2. The cell settling speed may vary depending on, for example, the cell size, morphology, or density. For example, the cell settling speed can be approximated by the Stoke's equation (1) below (Water Clarification Forum-Science and Technology-Basics of Settling Tank (http://water-solutions.jp/commentary/solid-liquid _separation/settling-pondbasics/)). The settling speed is proportional to the square of the particle diameter d, and large cells or aggregates settle rapidly. In contrast, the settling speed for small particles, such as debris, is slow.

$$v = (\rho_S - \rho_W)g/18\eta * d^2 \quad (1)$$

wherein:

v: the settling speed (m/s);
$\rho_s$: the particle density (kg/m$^3$);
$\rho_w$: the solution density (kg/m$^3$);
g: the gravitational acceleration (m/s$^2$);
$\eta$: the solution viscosity (Pa·s); and
d: the particle diameter.

Physical properties of the medium are assumed to be substantially equivalent to those of water herein. When the solution density $\rho_w$ is 997.8 kg/m$^3$, the solution viscosity $\eta$ is 0.89 mPa·s, the cell density $\rho_s$ is 1.05 g/ml, and the cell diameter d is 15 $\mu$m, the settling speed v would be 0.43 mm/min.

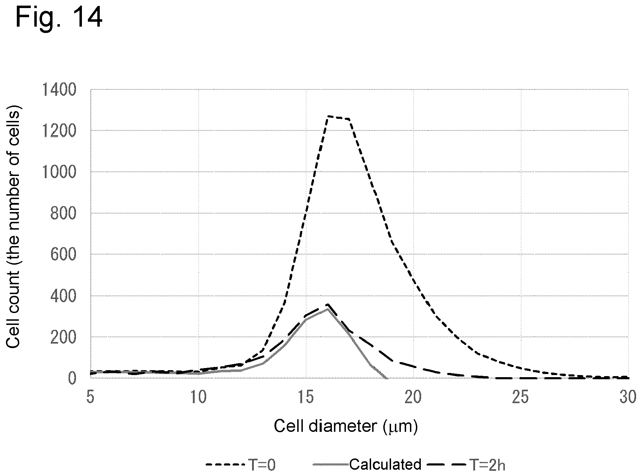

A cell culture fluid of CHO cells was introduced into a 15-ml centrifuge tube as a settling tank, and the cell counts in a clarified liquid layer (a 10-ml portion from the top of the 15-ml centrifuge tube) at T = 0 (the initial state) and at T = 2 hours were determined. Fig. 14 shows a chart demonstrating changes in cell distribution after cell settling in a clarified liquid layer in the settling tank. The vertical axis indicates the number of cells counted in a given amount of a cell culture fluid and the horizontal axis indicates the cell diameters. The "calculated" shown in Fig. 14 was obtained by, on the basis of the settling speed calculated by the Stoke's equation relative to cell diameters, calculating the proportion of the cells that reached the tube bottom to the cells that did not reach the tube bottom. The actual cell distribution at T = 2 hours is satisfactorily consistent with the change of the calculated distribution. This indicates that a rough estimation can be obtained by the Stoke's equation.

In fact, the viscosity and other properties of the cell culture fluid are changed with the elapse of culture time and also influenced by temperature changes. In addition, cell diameter distribution may vary depending on types of cells to be cultured. Thus, the cell count distribution in the settling tank relative to a latency of the gravitational settling may be determined via experimentation in advance as shown in Fig. 14.

When performing bleeding, changes in the cell density in the bioreactor can be determined in accordance with, for example, the formula (2) below. When the cell density is to be adjusted to a constant level, the bleed flow rate $F_b$ is determined in accordance with the formula (3) below (when the dying speed is sufficiently low relative to the cell growth speed) on the basis of the specific growth speed $\mu$ and the amount of the cell culture fluid Vw.

$$\frac{dX_v}{dt} = \mu * X_v - k * X_v - F_b/V_w * X_v \quad (2)$$

wherein:

Xv: the viable cell density (cells/ml);
$\mu$: the specific growth speed (/day);
k: the specific dying speed (/day);
Fb: the bleed flow rate (l/day); and
Vw: the amount of the cell culture fluid (1).

$$F_b = \mu * V_w \quad (3)$$

In general, the CHO cell doubling time is approximately 20 to 30 hours. Thus, the specific growth speed $\mu$ is 0.55 to 0.83 per day. Under high-density conditions in the stationary phase, however, the cell growth speed is often lowered by various factors, including nutrient depletion and increased growth inhibitory substances. In many cases, the specific growth speed $\mu$ may be in the range of 0.2 to 0.6 per day. Accordingly, the bleed flow rate $F_b$ is 0.2 to 0.6 Vw per day, and approximately 20% to 60% of the amount of the cell culture fluid Vw is discharged from the bioreactor as a bleed liquid per day.

In the present disclosure, a liquid flow (inflow/outflow) procedure of a cell culture fluid from the bioreactor to the settling tank is repeated. When a sequence is designated to be 30 minutes, the frequency of inflow into the settling tank would be 48 times (24 hours/0.5 hours) per day. If 60% of the cells flowed into the settling tank can be harvested, the bleed flow rate would be up to 0.6 Vw per day as described above. Since the number of instances by which cells flow into the settling tank is 48 per day, the volume of the settling tank necessary to store the cell culture fluid of such bleed flow rate would be 0.021 Vw (0.6 Vw/0.6/48). Thus, a sufficient volume of the settling tank would be approximately 2% of the amount of the cell culture fluid Vw in the bioreactor.

In the case of a bioreactor with a working volume of approximately 1 liter that has been extensively used for Jar culture, the volume of the settling tank would be approximately 20 ml. The cell settling speed deduced based on the Stoke's equation (1) is 0.43 mm/min. If 20 minutes in a sequence of 30 minutes are the settling latency, the cells would settle by 8.6 mm. When a settling tank is a cylinder with the volume of 20 ml, the height of 10 mm, and the radius of approximately 25 mm, cells with diameters of 15 $\mu$m would settle by 8.6 mm in the height of 10 mm during the latency of 20 minutes. That is, a majority of the cells would settle in a region accounting for 20% of the height from the bottom.

The position of the settling tank 2 is not particularly limited, provided that culture and cell separation/concentration (e.g., bleeding) performed in the bioreactor are not influenced. In the case of the settling tank with the size as described above, for example, the settling tank can be provided in a space in the upper part of the bioreactor (e.g., Fig. 3). When the settling tank is not provided with a temperature regulation mechanism, the settling tank may be provided in the bioreactor because of small temperature changes and small damages imposed on cells. In addition, the length of the tube communicating with the bioreactor can be reduced, and the influence on the cells remaining in the tube can be reduced. When the settling tank is provided outside the bioreactor, in contrast, a temperature regulation mechanism using a heater or other means may easily be provided, the radius of the settling tank can be increased, and the efficiency for concentration can be thus increased. While Fig. 3 shows an example of a constitution in which the settling tank 2 is provided in the bioreactor 30, the example does not limit the position of the settling tank 2 to be provided.

Tubes (1, 3, and 4) have sufficient diameters to allow cells and liquids to flow therethrough, including spaces in which liquids such as a cell culture fluid migrate. Diameters of tubes may not be the same with each other.

Positions where tubes (1, 3, and 4) are connected to the settling tank 2 affect the efficiency for concentration. In the concentration sequence described below, the tube 3 may be positioned in the lower part of the settling tank where cells settle. In some embodiments, the tube 3 may be positioned at the bottom of the settling tank. Since a fresh cell culture fluid flows into the settling tank 2 through the communicating tube 1, the communicating tube may be connected to the upper part of the settling tank that is not adjacent to the position of the tube 3 where the cell culture fluid is harvested, so as to avoid the cell culture fluid that has flowed into the settling tank from being harvested immediately (the constitution shown in Fig. 2). In the clarification sequence described below, the communicating tube 1 and the tube 4 may be provided at positions that are not adjacent to each other in the upper part of the settling tank (the constitution shown in Fig. 2). When harvesting a supernatant through the tube 4, accordingly, cells in the fresh cell culture fluid would settle before they reach the tube 4. Alternatively, a lateral-flow settling tank as exemplified in the modification example (5) below may be employed.

As the tubes 1, 3, and 4, the settling tank 2, the switching valve 5, the pump 6, the controller 7, the bioreactor 30, and the stirring rod 31, relevant components that have been generally used in cell culture can be used, and a person skilled in the art can adequately select such components. When a bioreactor with a working volume of approximately 1 liter (width: 120 mm; height: 220 mm) and a settling tank with a volume of approximately 20 ml (height: 10 mm; radius: 25 mm) are used, for example, a tube with an inner diameter of 2 mm and an outer diameter of 4 mm made of glass or SUS316 can be used.

Hereafter, modification examples of devices for liquid processing are described.

(1) Introduction of inclined plate

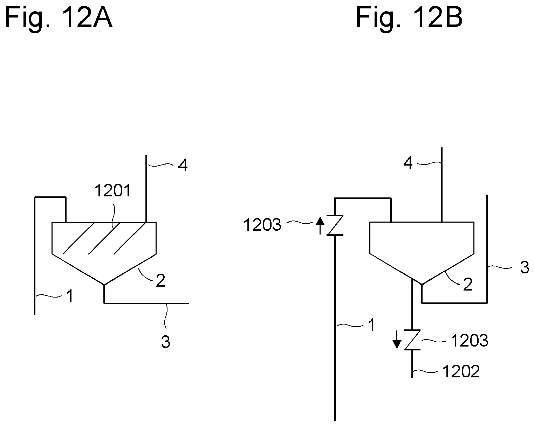

As shown in Fig. 12A, the inclined plate 1201 may be provided in the settling tank 2 so as to increase the efficiency for concentration in the settling tank. An inclined plate may not be particularly limited. For example, an inclined plate as described in Water Clarification Forum-Science and Technology-Basics of Settling Tank (indicated above) can be used.

(2) Increasing excretory tube

As shown in Fig. 12B, an additional excretory tube 1202 may be provided in the lower part or at the bottom of the settling tank 2. Thus, cells can be efficiently returned to the bioreactor through the excretory tube 1202. By increasing an excretory tube, efficiency of the clarification sequence is improved. In such a case, the check valve 1203 may be provided in the communicating tube 1 and/or the excretory tube 1202 for the cell culture fluid.

(3) Temperature regulation of settling tank

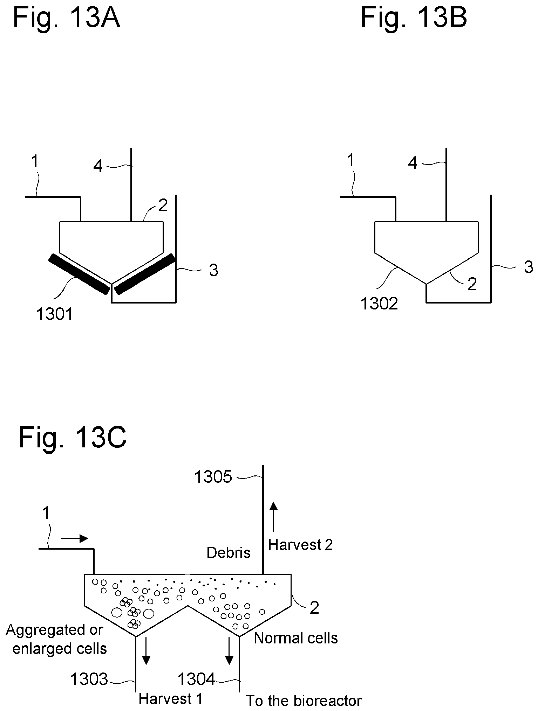

In order to adequately control temperature in the settling tank 2, a temperature regulation mechanism by means of, for example, the heater(s) 1301, may be added to the settling tank 2, as shown in Fig. 13A. Such constitution may be adopted when, in particular, the settling tank 2 is not provided in the bioreactor.

(4) Efficient oxygen incorporation

In order to prevent cells from oxygen depletion, as shown in Fig. 13B, the device is constituted to be capable of efficient oxygen incorporation into the settling tank 2 with the use of a means for oxygen incorporation, such as the oxygen permeation membrane 1302. For example, the entire plane of the settling tank 2 may be covered by the oxygen permeation membrane 1302, or the plane constituting the settling tank 2 may be made of the oxygen permeation membrane 1302.

(5) Enhanced function with the addition of pumps

As shown in Fig. 13C, the tubes 1303, 1304, and 1305 may be provided and pumps may be added thereto. Thus, normal cells are selectively returned to the bioreactor through the tube 1304, and debris, aggregated cells, and enlarged cells are discharged (harvested) from the bioreactor through the tube 1303 and the tube 1305. Thus, normal cells can be separated with higher efficiency.

(6) Integration of bioreactor with settling tank

The upper lid of the bioreactor may be integrated with the settling tank. Thus, the dimension of the settling tank can be more freely determined without using a space around the bioreactor. When a bioreactor is configured as a single-use container, in particular, operation can be simplified because of unnecessity of washing.

(Cell culture system)

Another aspect of the present disclosure provides a cell culture system. The cell culture system according to the present disclosure comprises:

a bioreactor is configured to accommodate a cell culture fluid containing cells;
a stirring device; and
the device for liquid processing according to the present disclosure.

The culture part in the cell culture system may not be particularly limited, provided that it comprises at least a bioreactor and cells can be cultured therein. A person skilled in the art can constitute an adequate culture part in accordance with, for example, the target cell type, the purpose of culture, or the necessary scale of culture. In an embodiment, the culture part can comprise a bioreactor and a culture medium bottle that is connected to the bioreactor and that supplies a medium to the bioreactor. A stirring device may not be particularly limited, provided that it can stir a cell culture fluid at an adequate speed without damaging cells. A stirring device may adequately be selected in accordance with, for example, the configuration and the size of the bioreactor to be used and the type of cells to be cultured. Examples of stirring devices that can be used include a stirring apparatus comprising a stirring blade or a stirring rod, an apparatus for stirring a cell culture fluid by shaking, rotating, or agitating the bioreactor (e.g., 2D wave bioreactor), and a pump for stirring or circulating a cell culture fluid.

In an embodiment, the cell culture system according to the present disclosure may further comprise a harvest tank which is configured to store a target product harvested from the cell culture fluid. In another embodiment, the cell culture system may comprise a bottle that is connected to the bioreactor to supply nutrients necessary for the bioreactor and a waste liquid bottle that is connected to the bioreactor to store the medium discarded from the bioreactor.

The cell culture system may comprise a control unit as a part of the system, which may be shared with the device for liquid processing, or the system may comprise an independent control unit. The control unit is configured to control at least one of cell supply, medium supply, bleeding, medium disposal, cell culture, and analysis of cell conditions. For example, the control unit is configured to control the volume of a cell culture fluid (e.g., the volume of the bleed liquid) to be supplied to the device for liquid processing based on the information concerning the cell growth speed in the bioreactor. Any control means that is known in the art can be used as the control unit. For example, a computer can be used.

For example, the device 20 for liquid processing according to an embodiment can be inserted into a space between the bioreactor and the bleed in the cell culture system as shown in Fig. 1B.

(Method of cell separation and/or cell concentration)

A further aspect of the present disclosure provides a method for separating and/or concentrating cells in a process of cell culture, comprising:

a step of transferring a cell culture fluid from a bioreactor to a settling tank;
a step of separating the cell culture fluid into a supernatant and a cell concentrate by the gravitational settling in the settling tank; and
a step of returning the supernatant to the bioreactor and harvesting the cell concentrate (the concentration sequence).

A further aspect of the present disclosure provides a method for separating and/or concentrating cells in a process of cell culture, comprising:

a step of transferring a cell culture fluid from a bioreactor to a settling tank;
a step of separating the cell culture fluid into a supernatant and a cell concentrate by the gravitational settling in the settling tank; and
a step of harvesting the supernatant and returning the cell concentrate to the bioreactor (the clarification sequence).

In an embodiment, at least one factor selected from the group consisting of the latency of the gravitational settling (also referred to as "a settling latency"), the time for transfer of the cell culture fluid, the time for transfer of the supernatant and/or the cell concentrate, the amount of the supernatant and/or the cell concentrate harvested, and the amount of the supernatant and/or the cell concentrate returned is/are calculated, on the basis of "the duration of a sequence" and "the amount of the processing liquid (e.g., the amount of bleeding)." "The duration of a sequence" may be designated by a user or a culture control apparatus. The term "the amount of the processing liquid (e.g., the amount of bleeding)" refers to the amount of the cell culture fluid transferred per unit time or the number of cells harvested per unit time.

In an embodiment, the liquid transfer speed may be controlled to suppress cells in the cell concentrate from floating when returning the supernatant to the bioreactor.

A flow example of a method of cell separation and/or concentration in a process of cell culture is described. The method of cell separation and/or concentration according to the present disclosure comprises: a concentration sequence of removing a cell culture fluid from a bioreactor and concentrating the cell density in the cell culture fluid; and/or a clarification sequence of removing a cell culture fluid from a bioreactor and clarifying the cell culture fluid.

In the present disclosure, both or either of the concentration sequence and the clarification sequence may be performed. The concentration sequence and the clarification sequence may be performed simultaneously. Alternatively, one of the concentration sequence and the clarification sequence may be performed at any point and the other may be performed at a different point in the process of cell culture.

(1) Concentration sequence

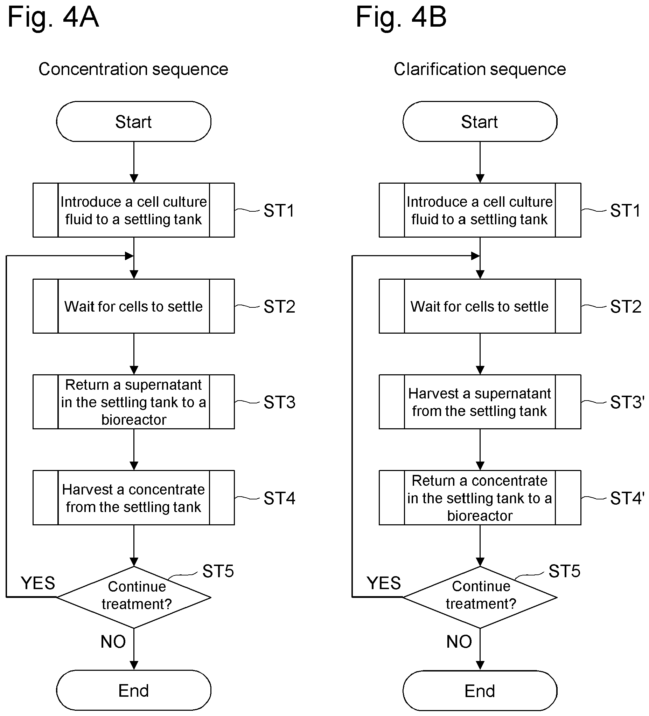

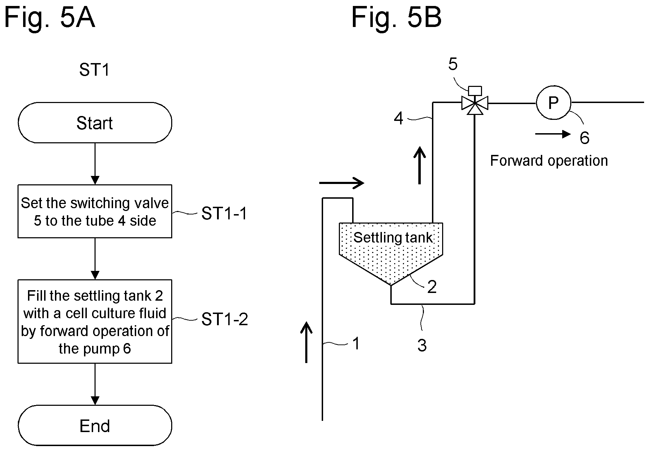

Fig. 4A shows a flow example of the concentration sequence. Also, Figs. 5A, 5B, 6A, 6B, 8A, 8B, 9A, and 9B each show a schematic diagram of an operation example of the device 20 for liquid processing (Fig. 2) according to an embodiment in the concentration sequence. This flow may be performed when performing bleeding in the process of cell culture but it can be applied to other processings.

The concentration sequence primarily comprises: a step of introducing a cell culture fluid into a settling tank (ST1); a step of waiting for cells to settle in the settling tank (ST2); a step of returning the supernatant in the settling tank to the bioreactor (ST3); and a step of harvesting a concentrate from the settling tank (ST4).

- ST1 (Fig. 4A, Figs. 5A and 5B): Introduce the cell culture fluid into the settling tank

The settling tank 2 may contain air in the beginning. Thus, the cell culture fluid is suctioned into the settling tank 2 with the use of the tube 4 connected to the upper part of the settling tank 2. In this case, suction is performed at a level greater than necessary so as to remove air from the tube 4. Specifically, the switching valve 5 is set to the tube 4 side (ST1-1) and the settling tank 2 is filled with the cell culture fluid by the forward operation of the pump 6 (ST1-2).

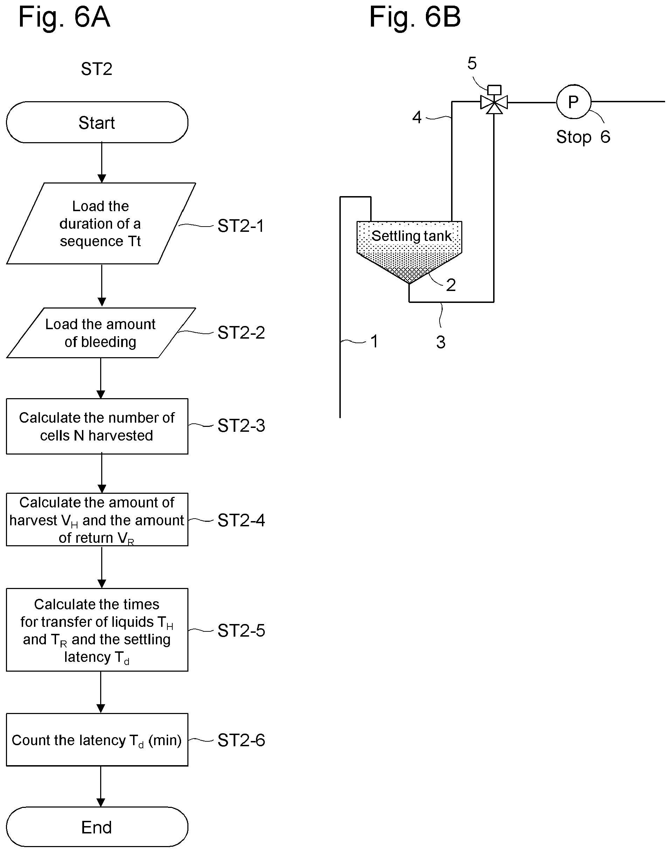

- ST2 (Fig. 4A, Figs. 6A and 6B): Wait for the cells in the settling tank to settle

The duration of a sequence Tt (excluding the duration of ST1) determined by the superior system or inputted by a user in advance is loaded (ST2-1). When the settling tank does not comprise the temperature or oxygen regulating mechanisms, the adequate time Tt may be determined in accordance with the host cell type or the cell line in view of changes in temperature, oxygen, or other conditions in the settling tank. Also, the amount of bleeding per unit time (the bleed flow rate $F_b$) is loaded (ST2-2). The bleed flow rate $F_b$ is determined by the superior system in accordance with the cell growth stage of the moment or updated by the user according to need.

Subsequently, the number of cells N to be harvested per sequence may be determined based on the duration of a sequence Tt, the bleed flow rate $F_b$, and the cell density Xv in the bioreactor (ST2-3). The cell count distribution in the settling tank corresponding to the settling latency $T_d$ may be determined via experimentation in advance or it may be determined with reference to the cell species exhibiting the similar distribution (refer to, for example, the description concerning Fig. 14 above or the description concerning Fig. 17 below). With the use of the distribution (since the accurate settling latency $T_d$ has not yet been determined at this point, an approximate latency $T_d'$, such as 0.7 Tt, is determined, and the distribution determined therefor is used) and the number of cells N, the amount of the concentrate to be harvested $V_H$ and the amount of the supernatant to be returned $V_R$ are determined (ST2-4). In addition, the liquid transfer times $T_H$ and $T_R$ may be determined based on $V_H$ and $V_R$ to determine the settling latency $T_d (T_t = T_d + T_H + T_R)$ (ST2-5). It is necessary to adequately determine the liquid transfer time $T_R$ when returning the supernatant to the bioreactor because the settled cells are floated when such time is short. For example, the cell floating speed can be approximated by the formula (4) below (Water Clarification Forum-Science and Technology-Basics of Settling Tank (indicated above)).

$$v_u = F_{in}/A \qquad (4)$$

wherein:

$v_u$: the particle floating speed;
$F_{in}$: the inflow speed into the bioreactor; and
A: the area of the settling tank.

It is sufficient if the floating speed $v_u$ of the settled cells is smaller than the settling speed v. When v is greater than $v_u$ ($v_u < v$), accordingly, the inflow speed $F_{in}$ into the bioreactor becomes smaller than the value obtained by multiplying the area A of the settling tank by the settling speed v ($F_{in} < A \times v$). In the case of the size of the settling tank on a 1-liter scale of the cell culture fluid described with reference to the device for liquid processing above, the area A of the settling tank is 1,963 mm$^2$ and the settling speed v is 0.43 mm/min. Accordingly, it is sufficient if the inflow speed $F_{in}$ into the bioreactor is 0.85 ml/min or smaller. If 50% of the volume (20 ml) of the settling tank is to be returned to the bioreactor, the liquid transfer time $T_R$ would be approximately 12 minutes.

The pump 6 may be stopped during the calculated settling latency $T_d$, and cells in the settling tank 2 then settle (ST2-6).

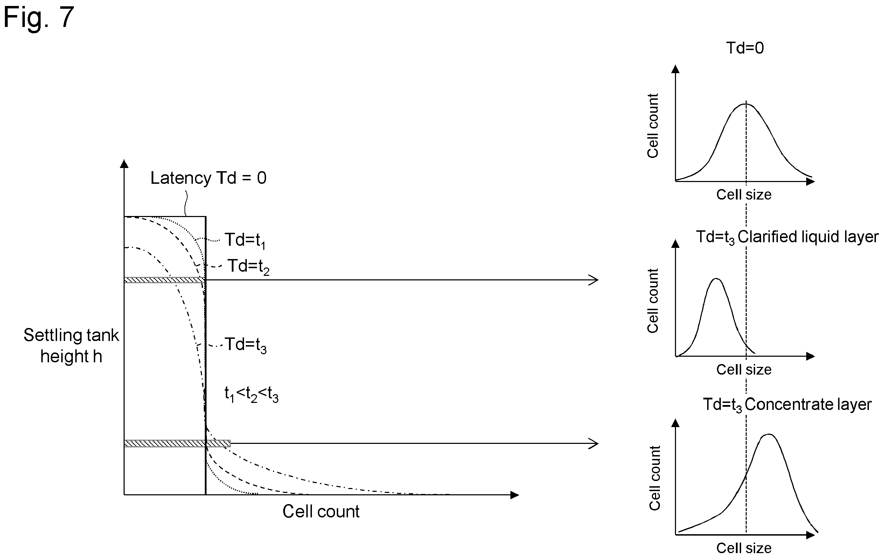

Fig. 7 shows a chart demonstrating the correlation between the settling latency and the number of cells settled when a cell culture fluid is separated by the gravitational settling in a settling tank. The way the cells settle with the elapse of time is represented in terms of the height h of the settling tank (the vertical axis) and the cell count (the horizontal axis). As the latency Td proceeds, the cells in the upper part migrate to the lower part in the settling tank. Since larger cells settle faster, as shown in the chart in the right side of Fig. 7, the cell diameter distribution of the clarified liquid layer (the upper part) shifts toward the negative side compared with the case when Td is 0 with the elapse of the settling latency Td (i.e., the number of large cells in the clarified liquid layer is small), but that of the concentrate layer (the lower part) shifts toward the positive side (i.e., the number of large cells in the concentrate layer is large). As described above, the cell count distribution in the settling tank corresponding to the settling latency $T_d$ may be determined via experimentation in advance or calculated with reference to the cell species exhibiting the similar distribution. Thus, the settling latency $T_d$, the amount of the concentrate to be harvested $V_H$, the amount of the supernatant to be returned $V_R$, and the like that are necessary for the number of cells to be harvested N can be determined.

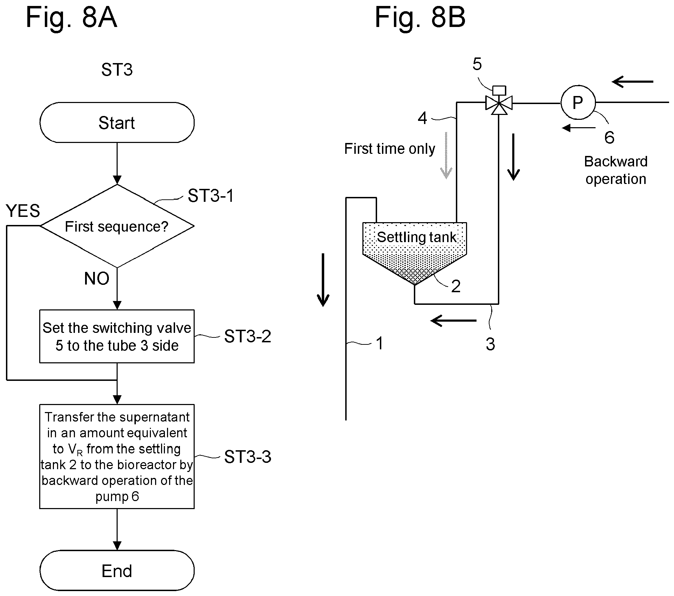

- ST3 (Fig. 4A, Figs. 8A and 8B): Return the supernatant in the bioreactor to the bioreactor

In view of the floating speed, the pump 6 may be operated backward at the predetermined liquid transfer speed, and the supernatant in the settling tank 2 is returned to the bioreactor in an amount equivalent to $V_R$ (ST3-3). In the first sequence, the tube 3 is filled with foams. Thus, the supernatant is returned through the tube 4 (ST3-1). In sequences other than the first sequence, the switching valve 5 is set to the tube 3 side (ST3-2). Thus, the concentrate once harvested would be returned to the settling tank in an amount equivalent to $V_R$.

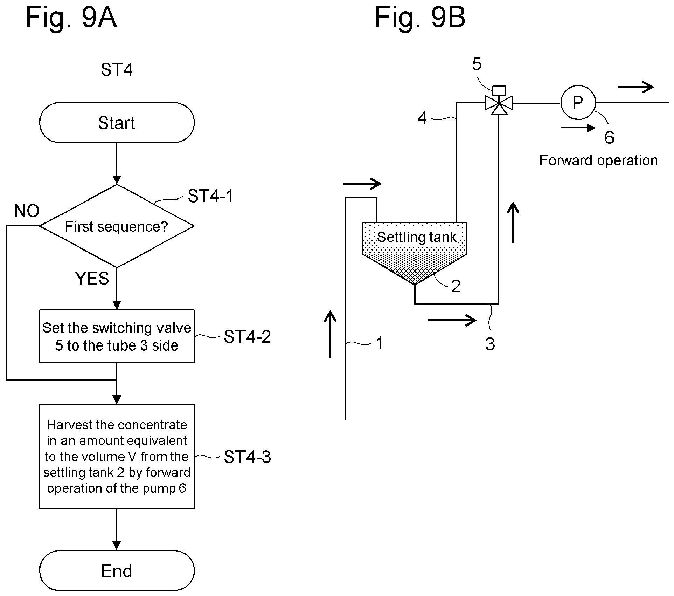

- ST4 (Fig. 4A, Figs. 9A and 9B): Harvest the concentrate from the settling tank

By the forward operation of the pump 6, the concentrate in an amount equivalent to the volume of the settling tank V may be harvested from the settling tank 2 (ST4-3). In the first sequence (ST4-1), the switching valve 5 is set to the tube 4 side by the procedure of ST3-1. Thus, the switching valve 5 is set to the tube 3 side (ST4-2).

Because of the procedure of ST3, the settling tank 2 contains a mixture of the concentrate $V_H$ obtained in the present sequence and the concentrate $V_R$ harvested in the prior sequence. If the concentrate in an amount V equivalent to the volume comprising both $V_H$ and $V_R$ ($V_H + V_R = V$) is harvested (ST4-3), the concentrate $V_H$ to be harvested in the present sequence would be harvested. By the operation of the pump in the procedure of ST4-3, in addition, a fresh cell culture fluid in an amount equivalent to V would be suctioned into the bioreactor 2.

- ST5 (Fig. 4A): Determine whether to continue the processing

When the processing is to be continued, the processing returns to the procedure of ST2 because the procedure of suctioning the cell culture fluid has been completed. When the processing is not continued, the concentration sequence is terminated.

As a result of the concentration sequence, the amount of the liquid to be processed (e.g., the amount of the bleed liquid) may be reduced, and the efficiency for harvesting the cell product via cell culture may be improved. If 80% of the cells flowed into the settling tank can be settled in a region equivalent to 20% lower part of the settling tank as a result of bleeding (i.e., when the amount of harvest is 0.2 V and the efficiency of harvesting is 80%), the cell density in the bleed liquid would be 4 times greater than the cell density Xv in the cell culture fluid ($0.8/0.2 \times Xv = 4$), and the amount of bleeding can be reduced to; i.e., cells can be concentrated to, 1/4 of the general level.

By performing the concentration sequence, a cell aggregate or enlarged cells can be efficiently discharged, and a good culture environment can be maintained. Since oxygen or nutrients are not sufficiently distributed inside the aggregate, cells that have become too large would die. Dead cells are degraded, proteases or nucleic acids are eluted from the cells, and debris also increase. Thus, the culture environment is deteriorated and cell death is further induced. The same applies to enlarged abnormal cells. If abnormal cells such as aggregates can be removed at an early stage, accordingly, a stable culture environment can be maintained.

(2) Clarification sequence

Fig. 4B shows a flow example of the clarification sequence. Also, Figs. Figs. 5A, 5B, 6A, 6B, 10A, 10B, 11A, and 11B each show a schematic diagram of an operation example of the device 20 for liquid processing (Fig. 2) according to an embodiment in the clarification sequence. This flow may be performed when performing bleeding in the process of cell culture but it can be applied to other processings.

The clarification sequence primarily comprises: a step of introducing a cell culture fluid into a settling tank (ST1); a step of waiting for cells to settle in the settling tank (ST2); a step of harvesting the supernatant from the bioreactor (ST3'); and a step of returning a concentrate in the bioreactor to the bioreactor (ST4').

- ST1 (Fig. 4A, Figs. 5A and 5B): Introduce the cell culture fluid into the settling tank

- ST2 (Fig. 4A, Figs. 6A and 6B): Wait for the cells in the settling tank to settle

ST1 and ST2 are the same as those in the concentration sequence. When the amount of harvest $V_H$ is to be calculated in ST2-4, it is calculated on the basis of the cell density in the concentrate in the concentration sequence. In the clarification sequence, it is calculated on the basis of the cell density in the supernatant.

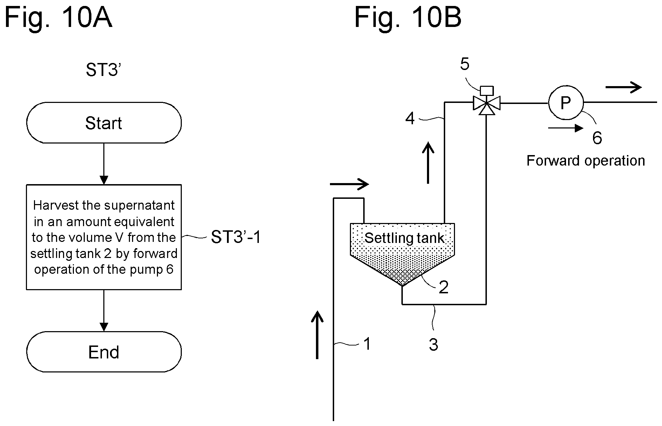

- ST3' (Fig. 4B, Figs. 10A and 10B): Harvest the supernatant from the bioreactor

The pump 6 may be operated forward at the predetermined liquid transfer speed to harvest the supernatant through the tube 4 in an amount equivalent to the total volume V of the settling tank 2 (ST3'-1). In this case, the cells that had settled in ST2 and the cells that had settled in ST3' are concentrated in the settling tank 2.

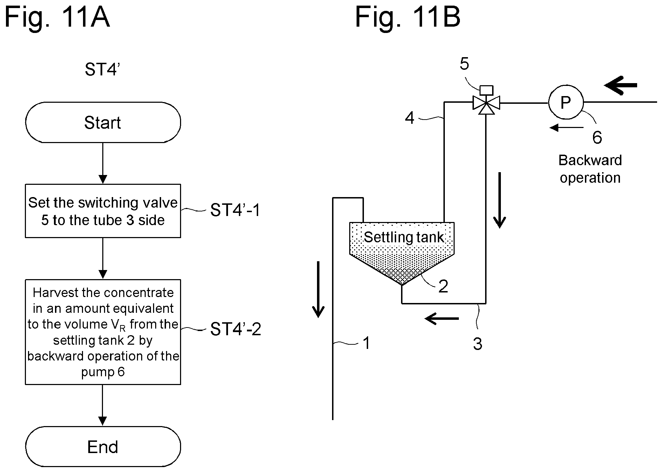

- ST4' (Fig. 4B, Figs. 11A and 11B): Return the concentrate in the settling tank to the bioreactor

The switching valve 5 is set to the tube 3 side (ST4'-1). By the backward operation of the pump 6, the concentrate in an amount equivalent to $V_R$ may be returned to the bioreactor (ST4'-2). In this case, the liquid transfer speed may be adjusted as quick as possible within the range in which shear stress on cells is acceptable, so as to return the cells concentrated in the lower part of the settling tank 2 in ST2 and ST3' to the bioreactor. Thus, the cell floating speed $v_u$ is increased, and the settled cells are mixed in the settling tank 2.

- ST5 (Fig. 4B): Determine whether to continue the processing

ST5 is the same as that in the concentration sequence.

In the clarification sequence, the cell density of the liquid to be processed (e.g., the bleed liquid) is lower than that of the cell culture fluid, and the amount of the processing liquid (e.g., the amount of bleeding) thus becomes larger than the general level. Accordingly, the volume of the settling tank 2 estimated for the device for liquid processing as described above (which is approximately 2% of the amount of the cell culture fluid) is not sufficient, and a settling tank of a larger size may be necessary. Thus, the clarification sequence is effective as a means of removing debris in a process involving a relatively low level of cell growth, or a means of intermittently removing debris in combination with the concentration sequence.

By performing the clarification sequence, debris in the cell culture fluid can be reduced, and a good culture environment can be maintained. In the production process of bio-pharmaceuticals, in particular, proteins or the like produced by cells are the target products, and excess cells should be removed. In the field of regenerative medicine, in contrast, cells are the products. In such a case, the method according to the present disclosure can be used as an efficient method for harvesting cells.

(Method for producing a target product via cell culture)

Another aspect of the present disclosure provides a method for producing a target product via cell culture. The method comprises:

a step of culturing cells that produce a target product in a bioreactor;
a step of implementing the method of cell separation and/or concentration; and
a step of harvesting the target product from the cell culture fluid in the bioreactor.

Production of useful substances via cell culture has been known in the art. By performing the method of cell separation and/or concentration according to the present disclosure in accordance with a conventional technique, a target product can be efficiently produced.

Cells that are commonly used for cell culture can be used without particular limitation. Examples of such cells include:

bacterial cells, such as cells of the genus *Escherichia,* such as *Escherichia coli,* and cells of the genus *Bacillus,* such as *Bacillus subtilis*;
animal cells, in particular, mammalian cells, such as COS, Vero, and Chinese hamster ovarian (CHO) cells;
insect cells, such as Sf9 and Sf21;
fungal cells, such as methanol-assimilable yeast (e.g., the genus *Komagataella* and the genus *Ogataea*), fission yeast (e.g., the genus *Schizosaccharomyces*), budding yeast (e.g., the genus *Saccharomyces*), and cells of the genus *Aspergillus,* such as *Aspergillus niger* and *Aspergillus oryzae;* and
plant cells, such as cells of plants such as tomatoes and tobaccos.

In the present disclosure, the term "target product" refers to a useful product that is expected to be obtained via cell culture. A target product may be an endogenous substance or a foreign substance of a cell. Examples of target products may include a protein, a sugar, a lipid, an alcohol, and a steroid, and a cell can be the target product. In some embodiment, a target product may be a protein. Examples of target proteins may include, but are not limited to, an antibody, an enzyme (e.g., phytase, amylase, glucosidase, cellulase, lipase, protease, glutaminase, peptidase, nuclease, oxidase, lactase, xylanase, trypsin, pectinase, and isomerase), protein A, protein G, protein L, fibroin, albumin, peptide, and fluoroprotein. In some embodiment, in particular, target proteins may be human and/or animal therapeutic proteins.

Specific examples of human and/or animal therapeutic proteins may include hepatitis B virus surface antigen, hirudin, an antibody or an antibody fragment (e.g., human antibody, humanized antibody, Fab antibody, and single chain antibody), serum albumin, human serum albumin, epithelial growth factor, human epithelial growth factor, insulin, growth hormone, erythropoietin, interferon, blood coagulation factor, granulocyte colony-stimulating factor (G-CSF), granulocyte macrophage colony-stimulating factor (GM-CSF), thrombopoietin, interleukin (e.g., IL-1, IL-6), tissue plasminogen activator (TPA), urokinase, leptin, and stem cell growth factor (SCF).

A target product (a protein, in particular) may be expressed inherently in a cell or may be expressed via genetic engineering in a cell. A method for expressing a target protein in a cell can be implemented by a method know in the art. For example, a recombinant vector comprising a gene encoding a target protein is constructed, the cell of interest is transformed with the recombinant vector, and a cell expressing a target protein can be thus prepared.

A step of cell culture may be performed in accordance with a method that is generally employed for culture of the cell to be used under the conditions in which a target product is produced. The "conditions in which a target product is produced" may include a medium suitable for production of a target product, culture temperature, a format of culture, and a duration of culture and such conditions may be adequately selected in accordance with a type of cells used. Culture can be performed in any medium, provided that such medium contains nutrients assimilable by cells. In some embodiments of the present disclosure, culture may be performed via perfusion culture, although the format of culture is not limited thereto.

Cell culture may be performed in either a naturally-occurring medium or a synthetic medium, provided that it contains a carbon source, a nitrogen source, inorganic salts, and the like assimilable by cells and is capable of efficiently performing cell culture. Examples of carbon sources that can be used may include carbohydrates, such as glucose, fructose, sucrose, and starch, organic acids, such as acetic acid and propionic acid, and alcohols, such as ethanol and propanol. Examples of nitrogen sources that can be used may include ammonium salts of inorganic or organic acids, such as ammonia, ammonium chloride, ammonium sulfate, ammonium acetate, and ammonium phosphate, other nitrogen-containing compounds, peptone, meat extracts, and corn steep liquor. Examples of inorganic substances that can be used may include monopotassium phosphate, dipotassium phosphate, magnesium phosphate, magnesium sulfate, sodium chloride, ferrous sulfate, manganese sulfate, copper sulfate, and calcium potassium.

In general, culture may be performed under aerobic conditions via agitation culture or aeration stirring culture at 32°C to 37°C for an adequate period of time. During culture, pH may be retained at 4.0 to 7.0. pH may be adjusted with the use of, for example, inorganic or organic acid, and an alkaline solution.

In the present disclosure, a method of cell separation and/or concentration is performed in the process of cell culture. The procedures to implement (the concentration sequence and/or the clarification sequence) and the time and the frequency to implement the procedure can be adequately determined in accordance with, for example, a type of cells to be used or a culture scale.

A target product (a protein, in particular) may be harvested and/or purified from the cell culture fluid obtained via cell culture as described above. The term "cell culture fluid" refers to, in addition to a culture supernatant, cultured cells or bacteria or disrupted cells or bacteria.

A target product (a protein, in particular) can be harvested and/or purified from the cell culture fluid by, for example, a conventional purification means. When a target product is a protein, for example, a target protein can be harvested and/or purified from the cell culture fluid by one of biochemical methods that are commonly used for protein isolation/purification or two or more thereof in adequate combinations. Examples of methods for protein isolation/purification may include gel filtration involving the use of Sephadex, Ultrogel, or Bio Gel, ion-exchange chromatography, electrophoresis involving the use of polyacrylamide gel, and fractionation involving the use of affinity chromatography or reversed-phase chromatography. Any of such methods may be performed by itself, or two or more thereof may be performed in adequate combination.

DESCRIPTION OF SYMBOLS

1: A communicating tube that allows a bioreactor to communicate with the settling tank 2
2: A settling tank
3: An excretory tube for a concentrate
4: An excretory tube for a supernatant
5: A flow path switching valve
6: A liquid transfer pump
7: A controller
20: A device for liquid processing
30: A bioreactor

31: A stirring rod
1201: An inclined plate
1202: An excretory tube
1203: A check valve
1301: A heater
1302: An oxygen permeation membrane
1303: An excretory tube
1304: An excretory tube
1305: An excretory tube

**Claims**

1. A device for use in liquid processing in a process of cell culture, comprising:

   a settling tank which is configured to store a cell culture fluid from a bioreactor;
   a communicating tube that allows the bioreactor to communicate with the settling tank;
   a first excretory tube for discharging a cell concentrate, which is connected to the settling tank;
   a pump; and
   a control unit,
   wherein, after the cell culture fluid is separated into a supernatant and the cell concentrate by the gravitational settling in the settling tank, the control unit is configured to control the pump to return the supernatant to the bioreactor through the communicating tube and to harvest the cell concentrate through the first excretory tube.

2. The device for use according to Claim 1, which further comprises a second excretory tube for discharging the supernatant, which is connected to the settling tank,
   wherein the control unit is configured to further control the pump to harvest the supernatant through the second excretory tube and to return the cell concentrate to the bioreactor through the communicating tube.

3. A device for use in liquid processing in a process of cell culture comprising:

   a settling tank which is configured to store a cell culture fluid from a bioreactor;
   a communicating tube that allows the bioreactor to communicate with the settling tank;
   a second excretory tube for discharging a supernatant, which is connected to the settling tank;
   a pump; and
   a control unit,
   wherein, after the cell culture fluid is separated into the supernatant and a cell concentrate by the gravitational settling in the settling tank, the control unit is configured to control the pump to harvest the supernatant through the second excretory tube and to return the cell concentrate to the bioreactor through the communicating tube.

4. The device for use according to Claim 3, which further comprises a first excretory tube for discharging the cell concentrate, which is connected to the settling tank,
   wherein the control unit is configured to further control the pump to return the supernatant to the bioreactor through the communicating tube and to harvest the cell concentrate through the first excretory tube.

5. The device for use according to Claim 2 or 4, which further comprises a flow path switching valve that connects the first excretory tube to the second excretory tube,
   wherein the control unit is configured to control switching of the flow path switching valve, forward operation and backward operation of the pump, and migration of the supernatant and the cell concentrate.

6. The device for use according to any one of Claims 1 to 5,

   wherein the communicating tube is connected to the upper part of the settling tank,
   the first excretory tube is connected to the lower part or the bottom of the settling tank, and/or
   the second excretory tube is connected to a site that is in the upper part of the settling tank and is not in contact with the communicating tube.

7. The device for use according to any one of Claims 1 to 6, wherein the settling tank is provided in the bioreactor.

8. The device for use according to any one of Claims 1 to 7, wherein the settling tank comprises a temperature regulation mechanism.

9. A cell culture system, comprising:

a bioreactor is configured to accommodate a cell culture fluid containing cells;
a stirring device; and
the device according to any one of Claims 1 to 8.

10. A method for separating and/or concentrating cells in a process of cell culture, comprising:

a step of transferring a cell culture fluid from a bioreactor to a settling tank;
a step of separating the cell culture fluid into a supernatant and a cell concentrate by the gravitational settling in the settling tank; and
a step of returning the supernatant to the bioreactor and harvesting the cell concentrate.

11. The method according to Claim 10, wherein, on the basis of the duration of a sequence, and the amount of the cell culture fluid transferred per unit time or the number of cells harvested per unit time, at least one factor is calculated, which is selected from the group consisting of the latency of the gravitational settling, the time for transfer of the cell culture fluid, the time for transfer of the supernatant and/or the cell concentrate, the amount of the cell concentrate harvested, and the amount of the supernatant returned.

12. The method according to Claim 10 or 11, wherein when returning the supernatant to the bioreactor, the speed for returning the supernatant is controlled so as to suppress the cells in the cell concentrate from floating.

13. A method for separating and/or concentrating cells in a process of cell culture, comprising:

a step of transferring a cell culture fluid from a bioreactor to a settling tank;
a step of separating the cell culture fluid into a supernatant and a cell concentrate by the gravitational settling in the settling tank; and
a step of harvesting the supernatant and returning the cell concentrate to the bioreactor.

14. The method according to Claim 13, wherein, on the basis of the duration of a sequence, and the amount of the cell culture fluid transferred per unit time or the number of cells harvested per unit time, at least one factor is calculated, which is selected from the group consisting of the latency of the gravitational settling, the time for transfer of the cell culture fluid, the time for transfer of the supernatant and/or the cell concentrate, the amount of the supernatant harvested, and the amount of the cell concentrate returned.

15. A method for producing a target product via cell culture, comprising:

a step of culturing cells that produce a target product in a bioreactor;
a step of implementing the method according to any one of Claims 10 to 14; and
a step of harvesting the target product from the cell culture fluid in the bioreactor.

Fig. 1A

Fig. 1B

Fig. 2

Fig. 3

# Fig. 4A

Concentration sequence

# Fig. 4B

Clarification sequence

# Fig. 5A

ST1

Start

Set the switching valve 5 to the tube 4 side ── ST1-1

Fill the settling tank 2 with a cell culture fluid by forward operation of the pump 6 ── ST1-2

End

# Fig. 5B

5

4

P

6

Forward operation

Settling tank

2

1       3

# Fig. 6A

ST2

```
┌─────────────────────────┐
│          Start          │
└─────────────────────────┘
             │
             ▼
      ╱──────────────╲
     ╱   Load the      ╲
    ╱   duration of a    ╲────── ST2-1
    ╲   sequence Tt      ╱
     ╲──────────────────╱
             │
             ▼
      ╱──────────────╲
     ╱ Load the amount ╲────── ST2-2
    ╱    of bleeding    ╱
     ╲────────────────╱
             │
             ▼
┌─────────────────────────┐
│ Calculate the number of │────── ST2-3
│     cells N harvested   │
└─────────────────────────┘
             │
             ▼
┌─────────────────────────┐
│  Calculate the amount of│
│ harvest $V_H$ and the amount│── ST2-4
│      of return $V_R$    │
└─────────────────────────┘
             │
             ▼
┌─────────────────────────┐
│   Calculate the times   │
│ for transfer of liquids $T_H$│
│  and $T_R$ and the settling │── ST2-5
│      latency $T_d$      │
└─────────────────────────┘
             │
             ▼
┌─────────────────────────┐
│ Count the latency $T_d$ (min)│── ST2-6
└─────────────────────────┘
             │
             ▼
┌─────────────────────────┐
│           End           │
└─────────────────────────┘
```

# Fig. 6B

Fig. 7

# Fig. 8A

ST3

```
      ┌─────────────┐
      │    Start    │
      └──────┬──────┘
             │
             ▼
YES      ◇─────────── ST3-1
◄────── First sequence?
      └──────┬──────┘
           NO│
             ▼
   ┌─────────────────┐
   │Set the switching valve│── ST3-2
   │ 5 to the tube 3 side  │
   └─────────┬───────┘
             │
             ▼
   ┌─────────────────┐
   │Transfer the supernatant│
   │in an amount equivalent │
   │to V_R from the settling│── ST3-3
   │tank 2 to the bioreactor by│
   │backward operation of the│
   │      pump 6     │
   └─────────┬───────┘
             │
             ▼
      ┌─────────────┐
      │     End     │
      └─────────────┘
```

# Fig. 8B

## Fig. 9A

ST4

## Fig. 9B

# Fig. 10A

ST3'

Start

Harvest the supernatant in an amount equivalent to the volume V from the settling tank 2 by forward operation of the pump 6 — ST3'-1

End

# Fig. 10B

5

4

P

6

Forward operation

Settling tank

2

1

3

# Fig. 11A

ST4'

Start

Set the switching valve 5 to the tube 3 side — ST4'-1

Harvest the concentrate in an amount equivalent to the volume $V_R$ from the settling tank 2 by backward operation of the pump 6 — ST4'-2

End

# Fig. 11B

5

4

P

6

Backward operation

Settling tank

2

1

3

# Fig. 12A

1201

4

2

1

3

# Fig. 12B

1203

4

2

3

1

1203

1202

## Fig. 13A

## Fig. 13B

## Fig. 13C

Fig. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019213497 A
- WO 2018159847 A

- WO 2015012174 A